# EUROPEAN PATENT SPECIFICATION

(11) **EP 1 418 871 B1**
(45) Date of publication and mention of the grant of the patent: **28.11.2007**
(21) Application number: 02739030.1
(22) Date of filing: 14.06.2002
(51) Int. Cl.: A61F 13/15, A41B 13/04

(54) **ABSORBENT PRODUCT**
SAUGFÄHIGES PRODUKT
PRODUIT ABSORBANT

(30) Priority: 03.07.2001 SE 0102397
(43) Date of publication of application: 19.05.2004
(73) Proprietor: SCA Hygiene Products AB, 405 03 Göteborg (SE)
(72) Inventor: SANDIN, Cécile, S-431 36 Mölndal (SE); NILSSON, Lennart, S-471 95 Skärhamn (SE)
(74) Representative: Olsson, Stefan
(86) International application number: PCT/SE2002/001149
(87) International publication number: WO 2003/003958

(56) References cited:
- WO-A1-00/61049
- WO-A1-01/13851
- GB-A- 2 112 267
- US-A1- 4 938 757

## Description

### TECHNICAL FIELD

The present invention relates to an absorbent product of pants-like shape, such as incontinence pants, nappy pants or sanitary towel pants, comprising an elastic waist portion, an absorbent element, which has a length and a width and is intended to at least cover the genitals of the wearer during use of the article, and a liquidtight outer layer which is intended to enclose the absorbent element on at least that side thereof which faces away from the wearer during use of the article. The invention also relates to a method of manufacturing such absorbent products of pants-like shape.

### BACKGROUND ART

Absorbent disposable products for taking up urine, faeces or menstrual blood have developed greatly since they came into more general use during the 1960s and 1970s. As they are disposable products, it is necessary that they can be manufactured and sold at a very low price. At the same time, it is important that the products function well and reliably. Good fit and comfort are also important characteristics. The first disposable nappies consisted of products in two parts, outer pants made of plastic, which were intended to be reused, and a rectangular absorbent insert which was disposable. The absorbent material in these inserts initially consisted of cellulose tissue. Later, better absorption materials made of what is known as fluff pulp made of cellulose were developed. The fit and comfort of these early nappies were poor. The products were unwieldy and uncomfortable for the wearer. Towards the end of the 1970s, the first complete disposable nappies arrived, that is to say nappies in which the absorption cores were integrated with a liquidtight outer layer. The absorption materials have developed and improved, which has resulted in the possibility of the absorption cores being adapted better to the anatomy of the wearer. Hourglass-shaped absorption cores with a narrower crotch portion between the two end portions are now predominant. The trend has also been towards increasingly thin products, which has been made possible by the inclusion of what are known as superabsorbent materials in the absorption body. There are many reasons why thinner and even smaller absorption bodies are desirable. A thinner, smaller absorption body is more comfortable and more discreet, which is especially important for adult incontinent wearers. A reduction in volume is also very important financially because the product then requires less storage space and is easier to transport and takes up less shelf space in shops. This is important for the financial management of the shops, and if a manufacturer can produce products requiring less space in the shops than the products of the competitors, this affords a not inconsiderable competitive advantage. Moreover, there is increased pressure from authorities, in particular as far as disposable articles are concerned, to use as little material as possible for the purpose of reducing the burden on the environment.

The smaller the absorption bodies become, the more important it becomes that the absorption bodies come to lie in the correct place directly in front of the genitals of the wearer and remain in place during use even when the wearer is very active and moves a great deal. The demands of consumers for discretion, comfort and reliable functioning are also increasingly exacting. Requirements for the absorption body to come to lie correctly when put on and then be retained in the correct place have therefore increased the need for good fixing of the product on the body and the need for very good adaptability to the body when the wearer moves, at the same time as requirements have increased for the product always to come to lie in the correct place when the article is put on the wearer. This has led to the development of what are known as nappy pants, which have elastic portions for improved fit and comfort and increased flexibility during movements of the wearer compared with conventional absorbent products.

An early patent publication relating to nappy pants of the disposable type is GB 2 112 267-A. However, this publication from 1983 discloses primitive nappy pants which did not become a commercial product. Not until the 1990s did absorbent products of pants-like shape and construction become a major commercial product. Pants-like products now exist in the form of nappies for infants and nappies for adults and to some extent sanitary towel pants for absorption of menstrual fluid. Previously commercially available nappy pants have been designed in principle like conventional nappies with a front portion and a rear portion and also an intermediate crotch portion, the front and rear portions being interconnected by a side seam between each leg opening and the waist opening of the pants. The nappy pants have been produced by plane nappy-like pieces being produced in a continuous web, the individual nappy pants being formed by nappy-like blanks being folded double and provided with said side seams to form nappy pants. These side seams project laterally from the finished product and are undesirable because they project and interfere with the fit of garments worn over the top. On account of their shape, they are visible through garments worn on top of the nappy pants and fitting closely around the body. They can also snag in clothing and even cause tears in nylon tights. Such projecting side seams can also chafe and give rise to pressure sores on wearers who spend a lot of time lying on their side. A pant of this type is described in US 4 938 757.

WO 00/61049 has proposed improved nappy pants, in which the projecting sides seams have been eliminated. In this construction, the side seams have been eliminated by virtue of elastic side portions extending continuously in one piece from the front portion of the nappy pants to their rear portion on both sides of the nappy pants. However, the nappy pants according to WO 00/61049 have a number of disadvantages. The nappy pants according to said publication have what is referred to as a chassis, which extends over the entire nappy pants and forms the front portion, the crotch portion and the rear portion and is most reminiscent of a conventional nappy, and also said elastic side panels which each overlap the chassis at both the front and the rear on the nappy pants. These overlapping portions do not serve any actual purpose on the finished product and are in fact undesirable because a lot of material is wasted, that is to say they are used for no other purpose than joining together. The overlapping, joined-together portions are less of a nuisance and less uncomfortable for the wearer than the projecting side seams on previously known nappy pants. Owing to their unfavourable positioning, particularly at the rear, the overlapping portions can still give rise to a risk of chafing and back sores on wearers who spend a lot of time lying on their back. Another disadvantage of the nappy pants according to WO 00/61049 is that said chassis is relatively large and, as this portion is relatively rigid at least in comparison with the elastic side portions of the nappy pants, the nappy pants as a whole are not as adaptable to the body of the wearer but there is a risk that the nappy pants will be displaced from their optimum position in relation to the body when a wearer lies in bed and moves. In particular the rigid rear portion of the nappy pants can be displaced and pull both the crotch portion and the front portion from their optimum positions directly in front of the genitals of the wearer because the front and crotch portion are essentially rigidly joined together in one piece with the rear portion.

### DISCLOSURE OF INVENTION

By means of the present invention, an improved absorbent product of pants-like shape has been produced.

A product of the type referred to in the introduction is to this end characterized in that said elastic waist portion is made from an elastic first piece which is essentially rectangular in the extended state and is intended to surround partly the trunk of the wearer and to form the rear portion and side portions of the pants-like product, in that a second piece forming part of the product is designed to form the front portion and crotch portion of the pants-like product, in that said second piece is elongate with two opposite end edges and two opposite longitudinal edges, in that the width of the second piece is, at least in the crotch portion, smaller than the length of the first piece, in that the second piece is arranged with its longitudinal direction at right angles in relation to the longitudinal direction of the first piece and is connected by a first end portion to one longitudinal edge portion of the first piece, centrally thereon, in that one end portion of the first piece is connected to a first side edge portion of the second piece, and in that the other end portion of the first piece is connected in a corresponding manner to a second side edge portion of the second piece, in addition to which the absorbent element is in its entirety arranged on the second piece.

By virtue of the fact that the entire rear portion as well is elastic and, together with the elastic side portions, forms a single continuous elastic first piece, the pants as a whole are more adaptable to body movements. Local irregularities are taken up and smoothed out by the continuous elastic piece and are not transferred to the more rigid parts of the front portion and crotch portion located directly in front of the genitals of the wearer. Compared with conventional nappies and previously known absorbent products of pants-like shape, the product according to the present invention affords superior fit and comfort. Most of the pants-like garment is completely smooth. The seams required are arranged at the transition between the front portion and the first piece and at the transition between the first and the second piece in the crotch portion. These seams come to lie in places which are not subjected to any significant pressure during use of the product, and there is less risk of chafing and pressure sores caused by seams on the pants-like product.

The design of the absorbent product according to the invention in only two part pieces, where the absorbent element is in its entirety located on the second part piece, affords greater freedom of choice with regard to manufacturing method compared with previously known products of pants-like shape.

According to one embodiment, the invention is characterized in that the length of the second piece is greater than the width of the first piece, and in that the projecting portion of the second piece formed by the length difference is in its entirety located below that side edge of the first piece which is the lower one during use of the product, and there forms the crotch portion. According to one embodiment, the invention is in this connection characterized in that said projecting portion has a smaller width than the remainder of the second piece.

According to another embodiment, the invention is characterized in that the absorbent element extends in its longitudinal direction over the entire crotch portion and a little way up over the front portion in the direction of that side edge of the first piece which is the upper one during use of the product.

According to another embodiment, the invention is characterized in that the absorbent element is arranged so as to extend with its lower end portion only a little way over the crotch portion and over less than half the length of the crotch portion formed by said projecting portion. According to one embodiment, the invention is in this connection characterized in that the absorbent element tapers in the direction of the crotch portion and is essentially triangular in plane form.

According to an embodiment in which the absorbent element extends over the crotch portion, the invention is characterized in that the absorbent element in a plane state with its side edge portions forms a concave shape in the crotch area.

According to another embodiment, the invention is characterized in that the second piece includes a liquid-permeable inner layer and said liquidtight outer layer, and in that the absorbent element is arranged between said inner and outer layers, the inner and outer layers extending in the lateral direction and longitudinal direction outside the absorbent element and being interconnected there

According to one embodiment, the invention is characterized in that said connected side edge portions and end portions of said first and second pieces are, before connection, arranged in an overlapping manner, with the inside of an overlapping portion being arranged against the outside of an overlapped portion.

A method of manufacturing a product of the type mentioned in the introduction is characterized according to the invention in that said elastic waist portion is made from an elastic first piece which is essentially rectangular in the extended state and is intended to surround partly the trunk of the wearer and to form the rear portion and side portions of the pants-like product, in that a second piece forming part of the product is designed to form the front portion and crotch portion of the pants-like product, in that said second piece is elongate with two opposite end edges and two opposite longitudinal edges, in that the width of the second piece is, at least in the crotch portion, smaller than the length of the first piece, in that the second piece is arranged with its longitudinal direction at right angles in relation to the longitudinal direction of the first piece and is connected by a first end portion to one longitudinal edge portion of the first piece, centrally thereon, in that one end portion of the first piece is connected to a first side edge portion of the second piece, and in that the other end portion of the first piece is connected in a corresponding manner to a second side edge portion of the second piece. According to one embodiment, the method is in this connection characterized in that the absorbent element is in its entirety arranged on the second piece forming part of the product before said piece is connected to said first piece.

### BRIEF DESCRIPTION OF DRAWINGS

The invention will be described in greater detail below with reference to illustrative embodiments shown in the accompanying drawings, in which
- Figure 1: shows diagrammatically a phase of the construction of an absorbent product according to a first embodiment;
- Figure 2: shows diagrammatically in plane form an assembled product according to the first embodiment;
- Figure 3: shows the product according to Figure 2 in perspective;
- Figure 4: shows a section along the line IV-IV in Figure 1;
- Figure 5: shows diagrammatically a phase of the construction of an absorbent product according to a second embodiment;
- Figure 6: shows in plane form an assembled product according to the second embodiment, and
- Figure 7: shows in plane form an assembled product according to a third embodiment.

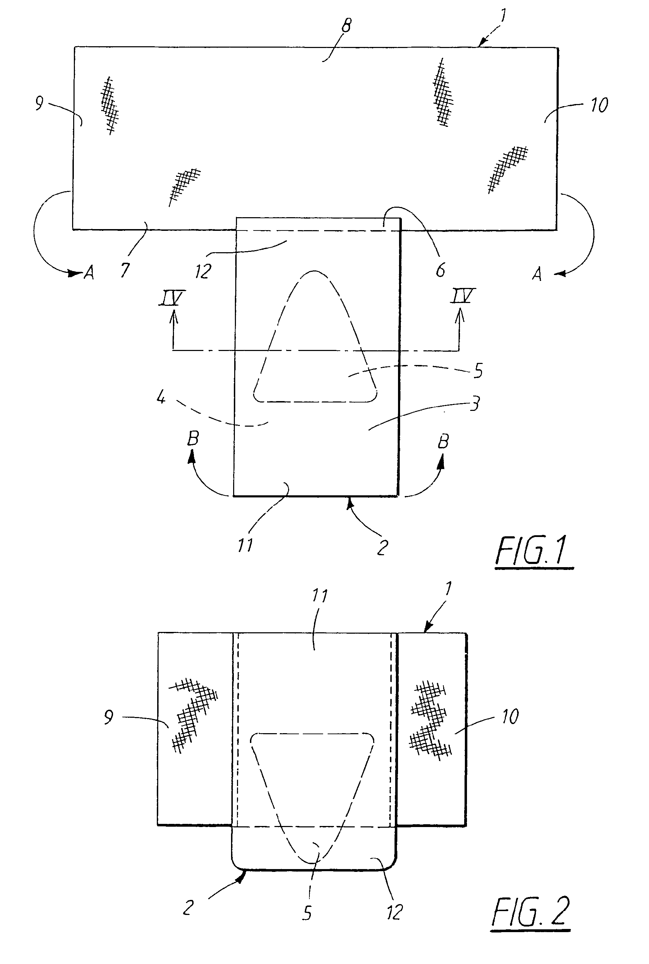

As can be seen from Figure 1, the product in the embodiment according to Figure 1 comprises a first piece 1. This piece is elastically stretchable and is shown in Figure 1 in a plane and even extended state, in which the elastic piece 1 is essentially rectangular. The elastic first piece 1 can be made from conventional materials well-known to the person skilled in the art, such as woven elastic materials, elastic non-wovens or elastic films. The important factor is that the elastic piece is stretchable for a wearer when the product is put on, and that it fits closely with suitable tightness around the wearer during use of the article. The tightness is of course regulated by means of size and elastic stretchability.

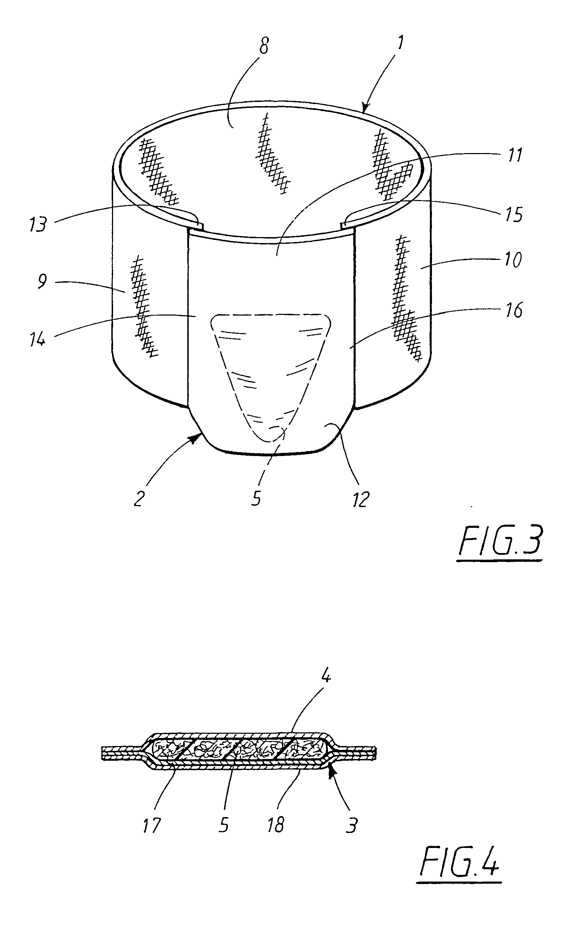

The product includes a second piece 2 which, as can be seen from Figures 1 and 4, consists of an outer layer 3, an inner layer 4 and an absorbent element 5 arranged between these. In the embodiment in plane form shown in Figure 1, this element is essentially triangular. The material selected for the absorption element is not critical, but it can be chosen from among materials or material combinations well-known to the person skilled in the art. For example, the absorbent element can consist of cellulose fluff pulp with superabsorbent materials in powder or fibre form added in. The outer layer 3 can consist of, for example, a polyethylene film of conventional type for absorbent products. A liquidtight film in combination with an outer fibre layer is suitable if an absorbent product with a more textile-like appearance is desired. The inner layer 4 can be made from a liquid-permeable non-woven. The second piece 2 is elongate and is arranged with its longitudinal direction at right angles in relation to the longitudinal direction of the first piece 1. The second piece 2 is connected by one end portion 6 to one longitudinal edge portion 7 of the first piece, centrally thereon. The connection can be made by means of, for example, adhesive, thermal bonding or ultrasonic bonding.

In Figure 1, the arrows A and B illustrate how the first and second pieces are folded to form the absorbent product of pants-like shape shown in Figures 2 and 3. The elastic piece 1 is folded in according to the arrows A shown to form the rear portion 8 and side portions 9, 10 of the product, while the second piece is folded upwards according to the arrows B to form the front portion 11 and crotch portion 12 of the product.

As can be seen most clearly from Figure 3, one end portion 13 of the first piece is arranged so as to be overlapped a little by a first side edge portion 14 of the second piece, and the other end portion 15 of the first piece is in a corresponding manner arranged so as to be overlapped a little by a second side edge portion 16 of the second piece. These overlapping portions are interconnected by means of, for example, adhesive, thermal bonding or ultrasonic bonding. Alternatively the front portion and the crotch portion can be arranged detachably along said overlapping portions 13, 14 and 15, 16. Such a detachable connection can be made by means of, for example, hook means (not shown), as a result of which the pants-like product can be opened and subsequently reclosed with the same fit and tightness.

In Figure 4, the thickness of the second piece has been exaggerated for the sake of clarity. The figure shows an outer layer 3 consisting of a laminate of a plastic film 17 and a non-woven layer 18 arranged outside. The inner layer 4 can consist of a liquid-permeable non-woven of a type well-known to the person skilled in the art.

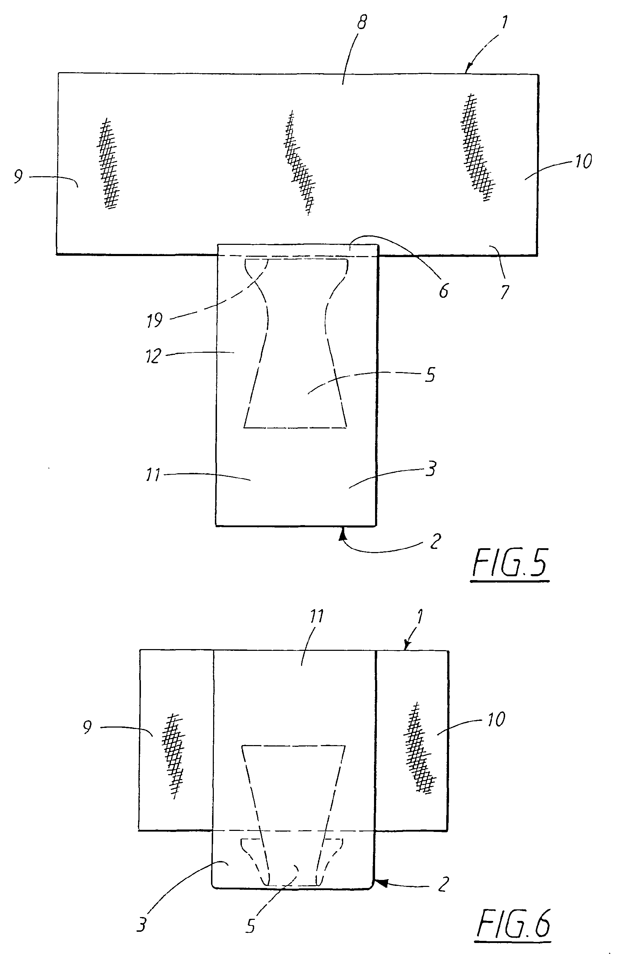

In the embodiment shown in Figures 5 and 6, those parts corresponding to the same parts in the embodiment according to Figures 1-4 have been provided with the same reference numbers. The only difference between the embodiment according to Figures 5 and 6 and that described above is the shape and extent of the absorbent element 5. In this second embodiment, the absorbent element 5 is hourglass-shaped in plane form, as can be seen most clearly from Figure 5, the narrower portion being intended to be arranged in the crotch of the wearer during use of the product. The upper end edge 19 of the absorbent element 5 is located directly adjacent to the lower edge portion 7 of the first elastic piece, which means that the absorbent element according to this second embodiment extends over the entire crotch area and a little way up over the front portion 11, as can be seen from Figure 6.

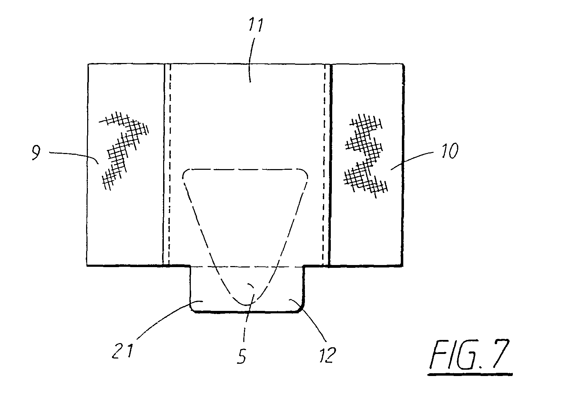

Figure 7 shows a third embodiment which is slightly modified in relation to the first embodiment. The only difference is that a part piece 21 of the second piece is made narrower than the remainder of the second piece, in which way a pants-like product with a narrower crotch portion is obtained, as can be seen from Figure 7.

The invention is not limited to the illustrative embodiments described above, but a number of modifications are possible within the scope of the patent claims below.

## Claims

1. Absorbent product of pants-like shape, such as incontinence pants, nappy pants or sanitary towel pants, comprising an elastic waist portion, an absorbent element (5), which has a length and a width and is intended to at least cover the genitals of the wearer during use of the article, and a liquidtight outer layer (3) which is intended to enclose the absorbent element on at least that side thereof which faces away from the wearer during use of the article, **characterized in that** said elastic waist portion is made from an elastic first piece (1) which is essentially rectangular in the extended state and is intended to surround partly the trunk of the wearer and to form the rear portion (8) and side portions (9, 10) of the pants-like product, **in that** a second piece (2) forming part of the product is designed to form the front portion (11) and crotch portion (12) of the pants-like product, **in that** said second piece (2) is elongate with two opposite end edges and two opposite longitudinal edges, **in that** the width of the second piece is, at least in the crotch portion (12), smaller than the length of the first piece (1), **in that** the second piece (2) is arranged with its longitudinal direction at right angles in relation to the longitudinal direction of the first piece and is connected by a first end portion (6) to one longitudinal edge portion (7) of the first piece, centrally thereon, **in that** one end portion (13) of the first piece is connected to a first side edge portion (14) of the second piece, and **in that** the other end portion (15) of the first piece is connected in a corresponding manner to a second side edge portion (16) of the second piece, in addition to which the absorbent element (5) is in its entirety arranged on the second piece (2).

2. Absorbent product according to Claim 1, **characterized in that** the length of the second piece (2) is greater than the width of the first piece (1), and **in that** the projecting portion of the second piece formed by the length difference is in its entirety located below that side edge of the first piece which is the lower one during use of the product, and there forms the crotch portion (12).

3. Absorbent product according to Claim 2, **characterized in that** said projecting portion has a smaller width than the remainder of the second piece (2) (Fig. 7).

4. Absorbent product according to Claim 2 or 3, **characterized in that** the absorbent element (5) extends in its longitudinal direction over the entire crotch portion (12) and a little way up over the front portion (11) in the direction of that side edge of the first piece (1) which is the upper one during use of the product.

5. Absorbent product according to Claim 2 or 3, **characterized in that** the absorbent element (5) is arranged so as to extend with its lower end portion only a little way over the crotch portion (12) and over less than half the length of the crotch portion formed by said projecting portion.

6. Absorbent product according to Claim 5, **characterized in that** the absorbent element (5) tapers in the direction of the crotch portion (12) and is essentially triangular in plane form.

7. Absorbent product according to Claim 4, **characterized in that** the absorbent element (5) in a plane state with its side edge portions forms a concave shape in the crotch area.

8. Absorbent product according to any one of the preceding claims, **characterized in that** the second piece (2) includes a liquid-permeable inner layer (4) and said liquidtight outer layer (3), and **in that** the absorbent element (5) is arranged between said inner and outer layers, the inner and outer layers extending in the lateral direction and longitudinal direction outside the absorbent element (5) and being interconnected there.

9. Absorbent product according to any one of the preceding claims, **characterized in that** said connected side edge portions (13, 15) and end portions (14, 16) of said first and second pieces (1 and, respectively, 2) are, before connection, arranged in an overlapping manner, with the inside of an overlapping portion being arranged against the outside of an overlapped portion.

10. Method of manufacturing an absorbent product of pants-like shape, such as incontinence pants, nappy pants or sanitary towel pants, comprising an elastic waist portion, an absorbent element (5), which has a length and a width and is intended to at least cover the genitals of the wearer during use of the article, and a liquidtight outer layer (3) which is intended to enclose the absorbent element on at least that side thereof which faces away from the wearer during use of the article, **characterized in that** said elastic waist portion is made from an elastic first piece (1) which is essentially rectangular in the extended state and is intended to surround partly the trunk of the wearer and to form the rear portion (8) and side portions (9, 10) of the pants-like product, **in that** a second piece (2) forming part of the product is designed to form the front portion (11) and crotch portion (12) of the pants-like product, **in that** said second piece (2) is elongate with two opposite end edges and two opposite longitudinal edges, **in that** the width of the second piece (2) is, at least in the crotch portion (12), smaller than the length of the first piece (1), **in that** the second piece is arranged with its longitudinal direction at right angles in relation to the longitudinal direction of the first piece and is connected by a first end portion (6) to one longitudinal edge portion (7) of the first piece, centrally thereon, **in that** one end portion (13) of the first piece (1) is connected to a first side edge portion (14) of the second piece (2), and **in that** the other end portion (15) of the first piece (1) is connected in a corresponding manner to a second side edge portion (16) of the second piece.

11. Method according to Claim 10, **characterized in that** the absorbent element (5) is in its entirety arranged on the second piece (2) forming part of the product before said piece is connected to said first piece (1).

## Patentansprüche

1. Absorbierendes Produkt mit höschenähnlicher Form, wie beispielsweise Inkontinenzhöschen, Höschenwindeln oder Höschenbinden, umfassend einen elastischen Taillenabschnitt, ein absorbierendes Element (5), das eine Länge und eine Breite aufweist und dazu gedacht ist im Gebrauch des Gegenstandes wenigstens die Genitalien des Trägers zu bedecken, und eine flüssigkeitsdichte Außenlage (3), die dazu gedacht ist das absorbierende Element auf wenigstens der Seite davon, die im Gebrauch des Gegenstands vom Träger weg weist, zu umschließen, **dadurch gekennzeichnet, dass** der elastische Taillenabschnitt aus einem elastischen ersten Stück, das im gedehnten Zustand im Wesentlichen rechteckig ist und dazu gedacht ist den Rumpf des Trägers teilweise zu umgeben und den Hinterabschnitt (8) und die Seitenabschnitte (9, 10) des höschenähnlichen Produkts zu bilden, aufgebaut ist und dadurch, dass ein zweites Stück (2), das ein Teil des Produkts bildet, ausgestaltet ist, um den Vorderabschnitt (11) und den Schrittabschnitt (12) des höschenähnlichen Produkts zu bilden, dadurch, dass das zweite Stück (2) länglich mit zwei gegenüberliegenden Endkanten und zwei gegenüberliegenden Längskanten ist, dadurch, dass die Breite des zweiten Stücks wenigstens im Schrittabschnitt (12) kleiner ist als die Länge des ersten Stücks (1), dadurch, dass das zweite Stück (2) mit seiner Längsrichtung im rechten Winkel in Bezug auf die Längsrichtung des ersten Stücks angeordnet ist und durch dass ein erster Endabschnitt (6) mit dem ersten Längskantenabschnitt (7) des ersten Stücks zentral dazu verbunden ist, dadurch, dass ein Endabschnitt (13) des ersten Stücks mit einem ersten Seitenkantenabschnitt (14) des zweiten Stücks verbunden ist und dadurch, dass der andere Endabschnitt (15) des ersten Stück auf entsprechende Art und Weise mit einem zweiten Kantenabschnitt (16) des zweiten Stücks verbunden ist, wobei das absorbierende Element (5) zusätzlich in seiner Gesamtheit auf dem zweiten Stück (2) angeordnet ist.

2. Absorbierendes Produkt nach Anspruch 1, **dadurch gekennzeichnet, dass** die Länge des zweiten Stücks (2) größer ist als die Breite des ersten Stücks (1) und dadurch, dass der vorragende Abschnitt des zweiten Stücks, der durch den Längenunterschied ausgebildet ist, in seiner Gesamtheit unter der Seitenkante des ersten Stücks angeordnet ist, die während dem Gebrauch des Produkts die niedrigere ist und dort den Schrittabschnitt (12) bildet.

3. Absorbierendes Produkt nach Anspruch 2, **dadurch gekennzeichnet, dass** der vorragende Abschnitt eine geringere Breite als der Rest des zweiten Stücks (2) aufweist (Fig. 7).

4. Absorbierendes Produkt nach Anspruch 2 oder 3, **dadurch gekennzeichnet, dass** sich das absorbierende Element (5) in Längsrichtung über den gesamten Schrittabschnitt (12) und eine gewisse Strecke nach oben über den Vorderabschnitt (11) in Richtung der Seitenkante des ersten Stücks (1), die im Gebrauch des Produkts die obere ist, erstreckt.

5. Absorbierendes Produkt nach Anspruch 2 oder 3, **dadurch gekennzeichnet, dass** das absorbierende Element (5) derart angeordnet ist, dass es sich mit seinem unteren Endabschnitt nur eine geringe Strecke über den Schrittabschnitt (12) und über weniger als die Hälfte der Länge des Schrittabschnitts, der durch den vorragenden Abschnitt gebildet ist, erstreckt.

6. Absorbierendes Produkt nach Anspruch 5, **dadurch gekennzeichnet, dass** sich das absorbierende Element (5) in Richtung des Schrittabschnitts (12) verjüngt und im Wesentlichen in der ebenen Form dreieckig ist.

7. Absorbierendes Produkt nach Anspruch 4, **dadurch gekennzeichnet, dass** das absorbierende Element (5) in einem ebenen Zustand mit seinen Seitenkantenabschnitten eine konkave Form im Schrittbereich bildet.

8. Absorbierendes Produkt nach einem der vorstehenden Ansprüche, **dadurch gekennzeichnet, dass** das zweite Stück (2) eine flüssigkeitsdurchlässige Innenlage (4) und die besagte flüssigkeitsdichte Außenlage (3) umfasst und dadurch, dass das absorbierende Element (5) zwischen der Innen- und Außenlage angeordnet ist, wobei sich die Innen- und Außenlagen in der seitlichen Richtung und der Längsrichtung über das absorbierende Element (5) hinaus erstrecken und dort verbunden sind.

9. Absorbierendes Produkt nach einem der vorstehenden Ansprüche, **dadurch gekennzeichnet, dass** die verbundenen Seitenkantenabschnitte (13, 15) und Endabschnitte (14, 16) des ersten und zweiten Stücks (1, 2) vor dem Verbinden in überlappender Art und Weise angeordnet sind, wobei die Innenseite eines überlappenden Abschnitts gegen die Außenseite eines überlappenden Abschnitts angeordnet ist.

10. Verfahren zum Herstellen eines absorbierenden Produkts mit höschenähnlicher Form, wie beispielsweise eines Inkontinenzhöschens, einer Höschenwindel oder einer Höschenbinde, umfassend einen elastischen Taillenabschnitt, ein absorbierendes Element (5), das eine Länge und eine Breite aufweist und im Gebrauch des Gegenstands dazu gedacht ist wenigstens die Genitalien des Trägers zu bedecken und eine flüssigkeitsdichte Außenlage (3), die dazu gedacht ist, das absorbierende Element auf wenigstens der Seite davon, die im Gebrauch des Gegenstands vom Träger weg weist, zu umschließen, **dadurch gekennzeichnet, dass** der elastische Taillenabschnitt aus einem elastischen ersten Stück (1) gebildet ist, das im gedehnten Zustand im Wesentlichen rechteckig ist und dazu gedacht ist den Rumpf des Trägers zu umgeben und den Hinterabschnitt (8) und Seitenabschnitt (9, 10) des höschenähnlichen Produkts zu bilden, dadurch, dass ein zweites Stück (2), das einen Teil des Produkts bildet, ausgestaltet ist, um den Vorderabschnitt (11) und den Schrittabschnitt (12) des höschenähnlichen Produkts zu bilden, dadurch, dass das zweite Stück (2) länglich mit zwei gegenüberliegenden Endkanten und zwei gegenüberliegenden Längskanten ist, dadurch, dass die Breite des zweiten Stücks (2) wenigstens im Schrittabschnitt (12) geringer ist als die Länge des ersten Stücks (1), dadurch, dass das zweite Stück mit seiner Längsrichtung im rechten Winkel in Bezug auf die Längsrichtung des ersten Stücks angeordnet ist und durch einen ersten Endabschnitt (6), der mit einem Längskantenabschnitt (7) des ersten Stücks zentral dazu verbunden ist, dadurch, dass ein Endabschnitt (13) des ersten Stücks (1) mit einem ersten Seitenkantenabschnitt (14) des zweiten Stücks (2) verbunden ist und dadurch, dass der andere Endabschnitt (15) des ersten Stücks (1) auf entsprechende Art und Weise mit einem zweiten Seitenkantenabschnitt (16) des zweiten Stücks verbunden ist.

11. Verfahren nach Anspruch 10, **dadurch gekennzeichnet, dass** das absorbierende Element (5) in seiner Gesamtheit auf dem zweiten Stück (2), das einen Teil des Produkts bildet bevor das Stück mit dem ersten Stück (1) verbunden wird, angeordnet ist.

## Revendications

1. Article absorbant en forme de culotte tel qu'une couche contre l'incontinence du type culotte, une couche d'apprentissage de la propreté, ou une serviette hygiénique du type culotte comprenant une partie ceinture élastique, un élément absorbant (5), qui comporte une longueur et une largeur et qui est destiné à couvrir au moins les parties génitales de l'utilisateur pendant l'utilisation de l'article, et une couche extérieure (3) imperméable aux liquides qui est destinée à enfermer l'élément absorbant sur au moins le côté de celui-ci qui est orienté à l'écart de l'utilisateur pendant l'utilisation de l'article, **caractérisé en ce que** ladite partie ceinture élastique est constituée d'une première pièce élastique (1) qui est sensiblement rectangulaire à l'état étendu et qui est destinée à entourer partiellement le tronc de l'utilisateur et à former la partie arrière (8) et les parties latérales (9, 10) de l'article du type culotte, **en ce qu'**une deuxième pièce (2) faisant partie de l'article a une forme destinée à constituer la partie avant (11) et la partie d'entrejambe (12) de l'article du type culotte, **en ce que** ladite deuxième partie (2) a une forme oblongue, avec deux bords d'extrémité opposés et deux bords longitudinaux opposés, **en ce que** la largeur de la deuxième pièce est, au moins dans la partie d'entrejambe (12), inférieure à la longueur de la première pièce (1), **en ce que** la deuxième pièce (2) est agencée de telle manière que sa direction longitudinale forme un angle droit par rapport à la direction longitudinale de la première pièce, et qu'elle est reliée par une première partie d'extrémité (6) à une des parties de bord longitudinal (7) de la première pièce, dans la zone centrale de celle-ci, **en ce qu'**une des parties d'extrémité (13) de la première pièce est reliée à une première partie de bord latéral (14) de la deuxième pièce, et **en ce que** l'autre partie d'extrémité (15) de la première pièce est reliée de manière correspondante à une deuxième partie de bord latéral (16) de la deuxième pièce, en plus de quoi l'élément absorbant (5) est disposé dans sa totalité sur la deuxième pièce (2).

2. Article absorbant selon la revendication 1, **caractérisé en ce que** la longueur de la deuxième pièce (2) est supérieure à la largeur de la première pièce (1) et **en ce que** la partie en saillie de la deuxième pièce formée par la différence de longueur est dans sa totalité située sous celui des bords latéraux de la première pièce qui est situé le plus bas pendant l'utilisation de l'article et y forme la partie d'entrejambe (12).

3. Article absorbant selon la revendication 2, **caractérisé en ce que** ladite partie en saillie a une largeur inférieure à celle du reste de la deuxième pièce (2). (Figure 7).

4. Article absorbant selon la revendication 2 ou 3, **caractérisé en ce que** l'élément absorbant (5) s'étend dans sa direction longitudinale au-dessus de la totalité de la partie d'entrejambe (12) et est quelque peu au-dessus de la partie avant (11) dans la direction de celui des bords latéraux de la première pièce (1) qui est situé le plus haut pendant l'utilisation de l'article.

5. Article absorbant selon la revendication 2 ou 3, **caractérisé en ce que** l'élément absorbant (5) est agencé de manière à ne s'étendre par sa partie d'extrémité inférieure que légèrement au-dessus de la partie d'entrejambe (12) au-dessus de moins que la moitié de la longueur de la partie d'entrejambe formée par ladite partie en saillie.

6. Article absorbant selon la revendication 5, **caractérisé en ce que** l'élément absorbant (5) est effilé en direction de la partie d'entrejambe (12) et a une forme sensiblement triangulaire, vu en plan.

7. Article absorbant selon la revendication 4, **caractérisé en ce que** l'élément absorbant (5), vu dans le plan de ses parties bords latéraux, a une forme concave dans la zone d'entrejambe.

8. Article absorbant selon l'une quelconque des revendications qui précèdent, **caractérisé en ce que** la deuxième pièce (2) comprend une couche intérieure (4) perméable aux liquides et ladite couche extérieure (3) imperméable aux liquides, et **en ce que** l'élément absorbant (5) est agencé entre lesdites couches intérieure et extérieure, les couches intérieure et extérieure s'étendant à l'extérieur de l'élément absorbant (5) dans la direction latérale et dans la direction longitudinale et y étant reliées entre elles.

9. Article absorbant selon l'une quelconque des revendications qui précèdent, **caractérisé en ce que** lesdites parties de bord latéral reliées (13, 15) et les parties d'extrémité (14, 16) desdites première et deuxième pièces (1 et respectivement 2) sont, avant d'être reliées, agencées de manière à se chevaucher, le côté intérieur d'une partie chevauchante étant disposé contre le côté extérieur d'une partie chevauchée.

10. Procédé de fabrication d'un article absorbant de forme analogue à celle d'une culotte tel qu'une culotte contre l'incontinence, une couche d'apprentissage de la propreté ou une serviette hygiénique du type culotte, comprenant une partie ceinture élastique, un élément absorbant (5), qui a une longueur et une largeur et qui est destiné à couvrir au moins les parties génitales de l'utilisateur pendant l'utilisation de l'article, et une couche extérieure (3) imperméable aux liquides qui est destinée à enfermer l'article absorbant sur au moins le côté de celui-ci qui est orienté à l'écart de l'utilisateur pendant l'utilisation de l'article, **caractérisé en ce que** ladite partie ceinture élastique est constituée d'une première pièce élastique (1) qui est sensiblement rectangulaire à l'état étendu et qui est destinée à entourer partiellement le tronc de l'utilisateur et à former la partie arrière (8) et les parties latérales (9, 10) de l'article du type culotte, **en ce qu'**une deuxième pièce (2) faisant partie de l'article est conçue pour former la partie avant (11) et la partie d'entrejambe (12) de l'article du type culotte, **en ce que** ladite deuxième pièce (2) a une forme oblongue, comportant deux bords d'extrémité opposés et deux bords longitudinaux opposés, **en ce que** la largeur de la deuxième pièce (2) est, au moins dans la partie d'entrejambe (12), inférieure à la longueur de la première pièce (1), **en ce que** la deuxième pièce est agencée de telle manière que sa direction longitudinale forme un angle droit par rapport à la direction longitudinale de la première pièce et qu'elle est reliée par une première partie d'extrémité (6) à une des parties (7) de bord longitudinal de la première pièce, dans la zone centrale de celle-ci, **en ce qu'**une des parties d'extrémité (13) de la première pièce (1) est reliée à une première partie de bord latéral (14) de la deuxième pièce (2), et **en ce que** l'autre partie d'extrémité (15) de la première pièce (1) est reliée de manière correspondante à une deuxième partie (16) de bord latéral de la deuxième pièce.

11. Procédé selon la revendication 10, **caractérisé en ce que** l'élément absorbant (5) est disposé dans sa totalité sur la deuxième pièce (2) en constituant une partie de l'article avant que ladite pièce soit reliée à ladite première pièce (1).
